# EUROPEAN PATENT SPECIFICATION

(11) **EP 2 142 118 B1**
(45) Date of publication and mention of the grant of the patent: **19.02.2014**
(21) Application number: 07736836.3
(22) Date of filing: 04.05.2007
(51) Int. Cl.: A61B 17/42

(54) **UTERINE MANIPULATOR**
VORRICHTUNG ZUM MANIPULIEREN DES UTERUS
MANIPULATEUR UTÉRIN

(43) Date of publication of application: 13.01.2010
(73) Proprietor: Sofar SPA, 20060 Trezzano Rosa (MI) (IT)
(72) Inventor: DAMIANI, Alfredo Maria, I-20100 Milano (IT); MELGRATI, Luigi, I-20032 Cormano (MI) (IT); FRANZONI, Gianalfredo, I-20052 Monza (MI) (IT)
(74) Representative: Pistolesi, Roberto
(86) International application number: PCT/IT2007/000333
(87) International publication number: WO 2008/136024

(56) References cited:
- WO-A-94/00061
- WO-A-03/015643
- DE-U1- 20 110 921
- GB-A- 2 284 242
- US-A- 5 382 252
- US-A- 5 409 496

## Description

The present invention relates to a uterine manipulator, in particular for a hysterectomy, even more particularly for a laparoscopic hysterectomy, and the following description refers to this field of application for the sole purpose of simplifying disclosure thereof.

As is well known, laparoscopy is a technique extensively applied in surgery and diagnosis and consists in the visual observation of the interior of the abdominal cavity by means of a TV camera situated at the end of a slim probe which is inserted in the patient's abdominal cavity through a small incision in the region of the umbilical scar.

The interior of the abdominal cavity can therefore be seen on a TV screen.

Diagnostic laparoscopy refers to the situation where the operator only performs an examination, at most only moving the organs, and operative laparoscopy to the situation where the operator carries out an actual surgical operation.

In gynaecology very many operations can be performed by this method, from the enucleation of ovarian cysts to the removal of fibromas, removal of the uterus (hysterectomy) and the like.

In the case of operative laparoscopy other instruments are inserted through 2 or 3 small incisions which are made in the lower part of the abdomen.

More particularly, in the laparoscopic hysterectomy operation, in addition to the TV camera, a cutting instrument is inserted in the abdominal cavity through holes made in the iliac fossae, together with a clamp for keeping the organ to be operated in position.

In this type of operation a uterine manipulator is essential, being inserted in the uterus via the vagina, as adequate mobilisation of the uterus is required, ensuring the following actions:
- anteversion, retroversion, lateral movement and elevation of the uterus;
- identification and delimitation of the anterior and posterior folds and the lateral fornices;
- visualisation of the uterine artery and its isolation in relation to the ureter.

A number of uterine manipulators which perform the above movements for a laparoscopic hysterectomy are already known and used; the most recently produced and also advanced manipulator is the Clermont-Ferrand manipulator.

This manipulator comprises a rod which at one end has a handle with locking screw and at the other end a tiltable conical tip and an anatomical valve for exposing the vaginal fornices.

The manipulator also comprises a grip perpendicular to the rod of the instrument, which during use remains outside the vagina and is used to guide the movements of the manipulator, and a cylinder formed by three silicone seals, fitted on the rod for maintaining the pneumoperitoneum, that is to say for maintaining a gaseous atmosphere in the peritoneal cavity.

The manipulator is inserted, through the vagina, into the cervix; the grip is rotated around its axis so that the conical tip is screwed into the uterus. When the manipulator is fixed in the uterus, the movements of anteversion and retroversion and lateral and elevation movements of the uterus mentioned previously can be performed. A snap-engagement mechanism allows the manipulator to be locked in five positions which determine five consequent conditions of mobilisation of the uterus.

The grip is used to locate the fornices, pushing it forwards and manoeuvring it in the most appropriate manner.

Nevertheless the Clermont-Ferrand manipulator has disadvantages.

It is a device that is very expensive and complex to assemble after the frequent and necessary cleaning operations; moreover its weight is considerable (a few kilos) and it is therefore not very easy to handle and consequently fairly dangerous during use.

Insertion in the uterus is awkward and requires high dilation of the cervix (at least up to Hegar 9).

Therefore, only highly expert and well-trained operators are allowed to use the manipulator. The manipulator in question is in fact designed specifically for use in laparoscopic hysterectomy operations and can only be used as it is for other diagnostic and surgical needs.

WO 03/015643 teaches a manipulation rod according to the preamble of claim 12. DE 201 10 927 U1 discloses a uterine manipulator comprising a rod and a complementary unit according to claim 1.

The object of the present invention is to provide a uterine manipulator which is improved in terms of easy handling and versatility for overcoming the disadvantages of the prior art.

A further object is to provide a simplified manipulation member that can be used for various types of examination and mobilisation operations, which can be performed in vivo.

### SUMMARY OF THE INVENTION

The technical problem is solved by means of a uterine manipulator as defined in claim 1.

The mobilisation rod according to claim 12, considered singularly and independently, also constitutes the object of the invention.

The features and advantages of the invention will be made clearer by the following description hereinbelow of one of its embodiments, given by way of a non-limiting example with reference to the accompanying drawings.

### BRIEF DESCRIPTION OF THE DRAWINGS

- Figure 1A shows a sectioned view of a manipulator according to the invention.
- Figure 1B shows a perspective view of the manipulator of Figure 1A.

- Figure 1C shows the manipulator of Figures 1A and 1B broken down into its component parts.
- Figure 2 shows a sectioned view of a rod of the manipulator of the previous drawings.
- Figure 3 shows a sectioned view of the manipulator during three different operative stages, according to the invention.
- Figure 4A shows a sectioned view of a first portion of the manipulator.
- Figure 4B shows a detail of Figure 3A.
- Figure 5 shows an exploded view of a second portion of the manipulator, according to the invention.
- Figure 6 shows a first detail of Figure 4.
- Figure 7 shows a second detail of Figure 4.
- Figures 8a and 8b show a first sectioned view of a third portion of the manipulator in two different operative conditions, according to the invention.
- Figure 9 shows a perspective view of a detail of the third portion of the manipulator, according to the invention.
- Figure 10 shows a sectioned view of a portion of a complementary unit of the manipulator according to the invention.
- Figure 11 shows a schematic front view of a section of the abdominal cavity of a patient undergoing a laparoscopic hysterectomy.
- Figure 12 shows a schematic side view of a section of the abdominal cavity of a patient undergoing a laparoscopic hysterectomy.

### DETAILED DESCRIPTION

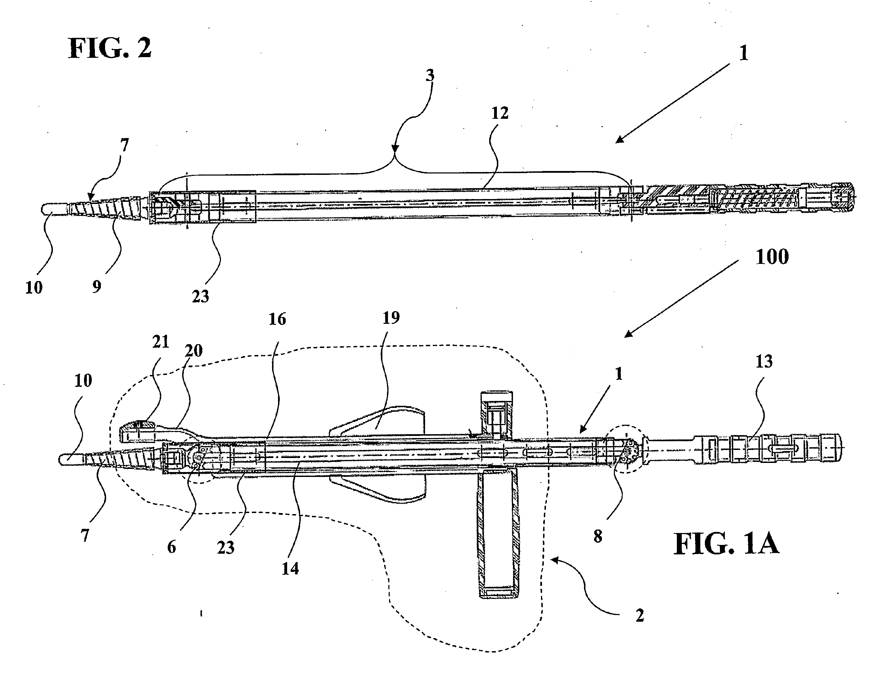

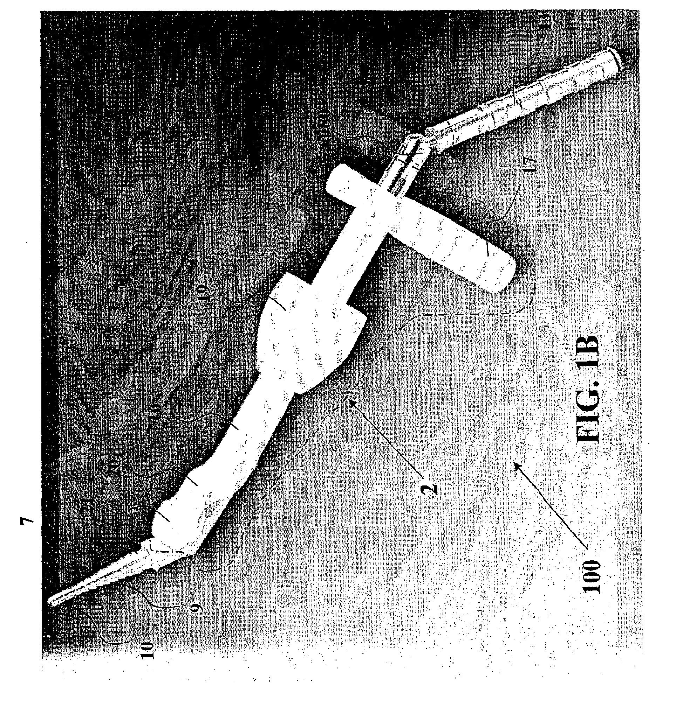

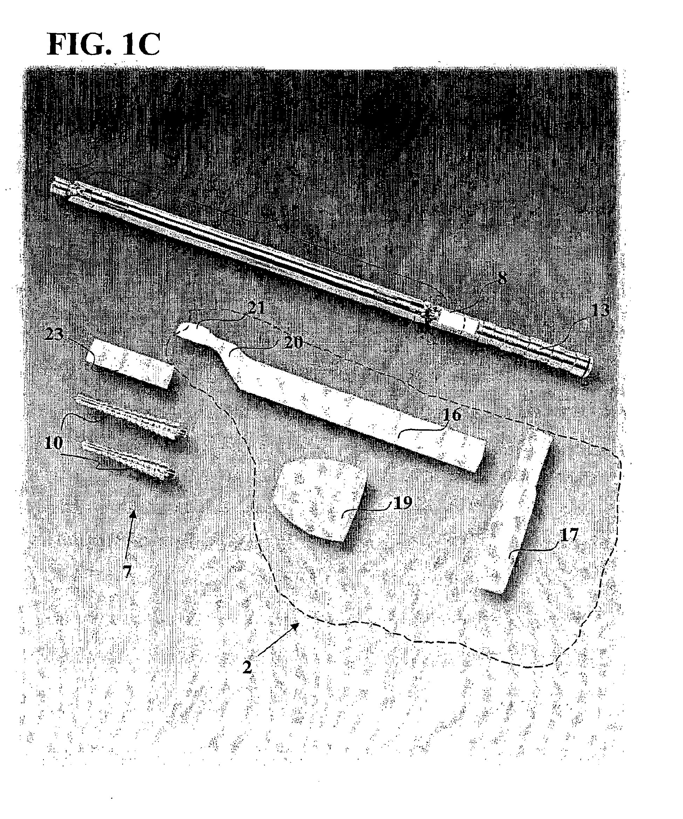

A uterine manipulator 100, shown in full in Figures 1A, 1B and, broken down into its component parts, in Figure 1C, comprises a mobilisation rod 1 and a complementary unit 2. The mobilisation rod 1 is shown in full and individually in Figure 2.

The mobilisation rod 1, referring in particular to Figures 2 and 1C and 3, comprises a central tubular body 3 at the ends of which a front joint 6 and a rear joint 8 are costrained, said joints allowing connection to a screw 7 and a first grip 13 respectively.

The screw 7 consists of a threaded body 9 and a rounded tip 10.

The threaded body 9 is preferably conical with saw teeth and the tip 10 is made of anodised aluminium.

The screw 7 establishes a mechanical connection between the mobilisation rod 1 and the uterus of the patient; the threaded body 9 is specially designed for anchoring to the neck of the uterus and for holding it to allow its mobilisation during the operation. Several interchangeable tips 10 of varying length are provided depending on the characteristics of the patient's uterus.

The central tubular body 3 is provided, near the joint 6, with slits for allowing internal cleaning thereof.

A flexible cap 23 with a hollow cylindrical shape, preferably made of silicone, covers the aforesaid slits during a hysterectomy operation and protects the part of the manipulator at the joint 6.

The central tubular body 3 is made up of a cylinder 12 which encloses an articulated quadrilateral structure 14; the cylinder 12 constitutes the external rigid support for connection between the screw 7 and the first grip 13 for controlling the manipulator, while the articulated quadrilateral 14 and a fork-shaped structure 40 connect and guarantee the reciprocal and interdependent movements of the first control grip 13 and the screw 7.

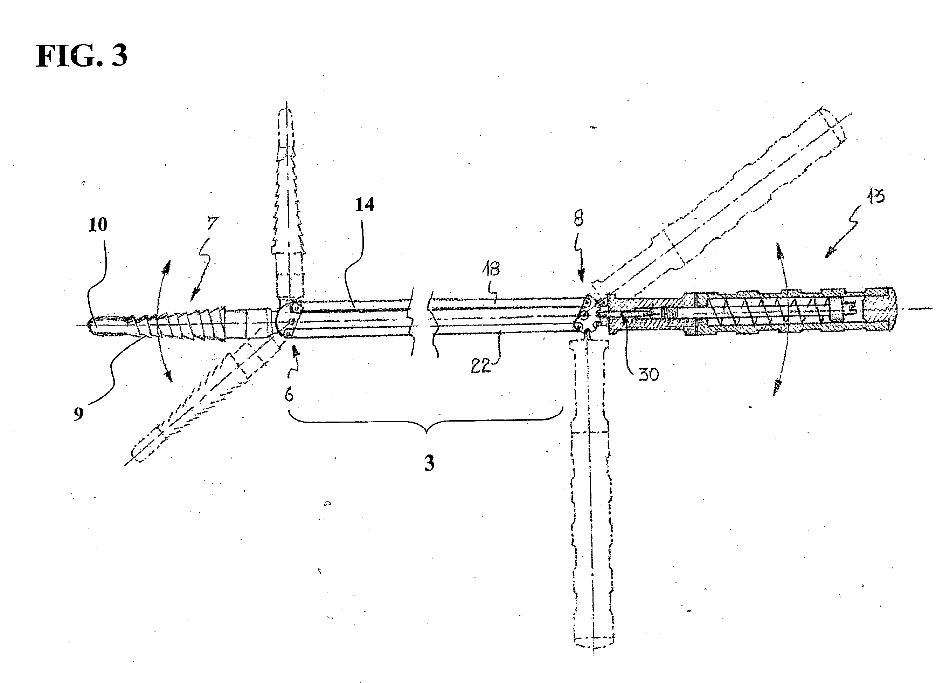

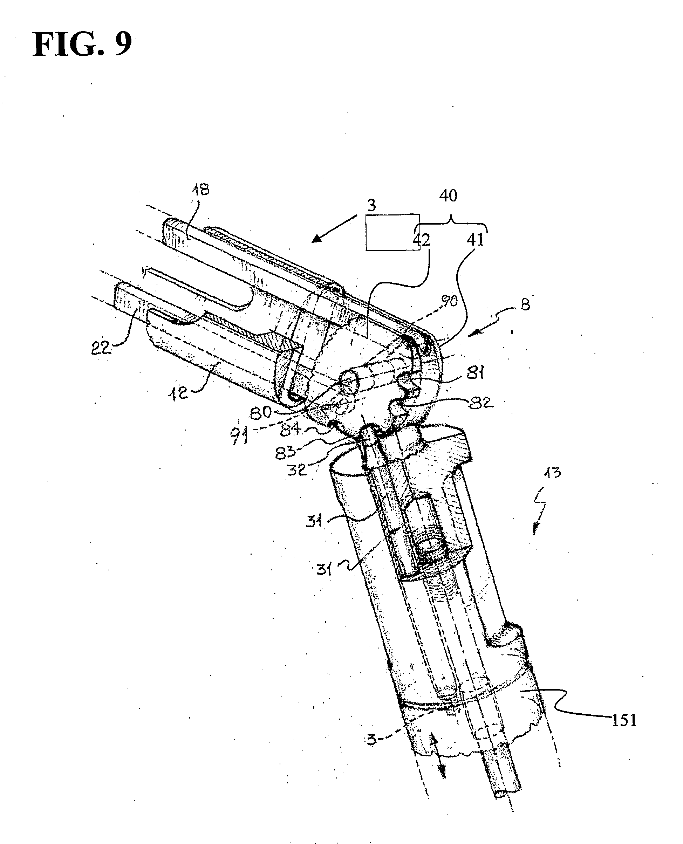

The fork-shaped structure 40 (shown in Figure 9) ensures the rotating connection between the control grip 13 and the central tubular body 3. This fork 40 has two branches 41 and 42, branch 41 of which acts as a support shoulder to allow relative rotation of the first control grip 13 in relation to the central tubular body 3, about the central pin 80, while the branch 42 consists of a half disk with notches 81, 82, 83 and 84, shown in detail in Figure 3B, which acts as a joint 8.

The notches are formed in different angular positions in relation to the first central pin 80. Naturally the number of notches shown in the figures is not limiting and can be varied during the design stage depending on the more or less stringent requirements for movement of the manipulator.

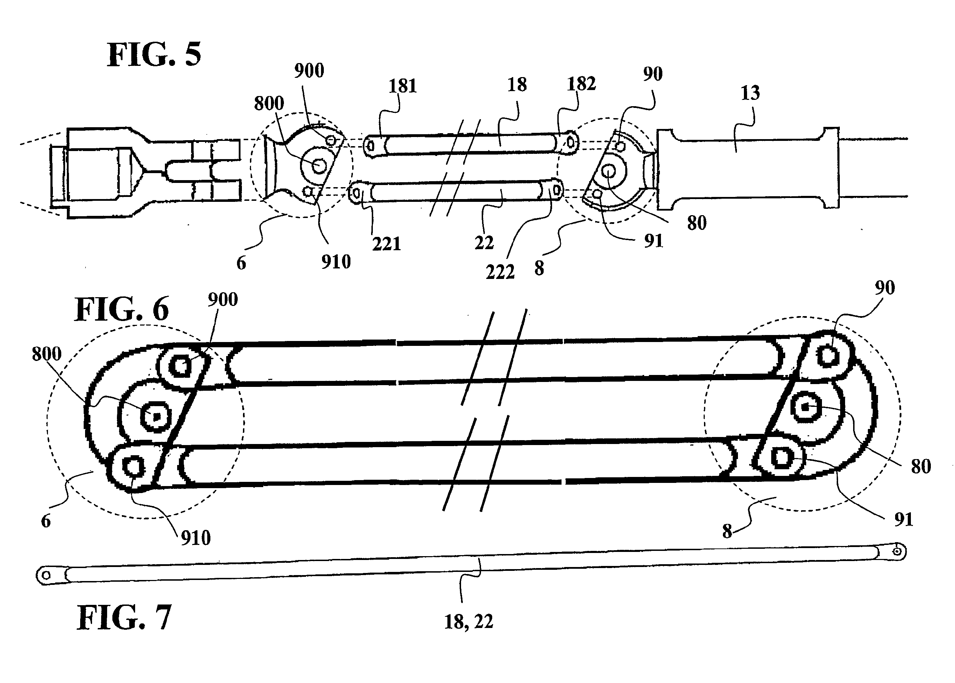

The joints 6, 8 are shown schematically, with various levels of detail, in Figures 1A, 3A, 8a and 8b and more accurately in Figures 5, 6 and 4B.

The quadrilateral 14 is attached between the two branches 41 and 42 and is formed by two parallel rods 18 and 22 of equal length (shown as coupled in Figures 5 and 6 and individually in Figure 7); on the side of the first control grip 13, these rods end with eyelets 182, 222 hinged, by means of respective first pins 90 and 91, to the joint 8.

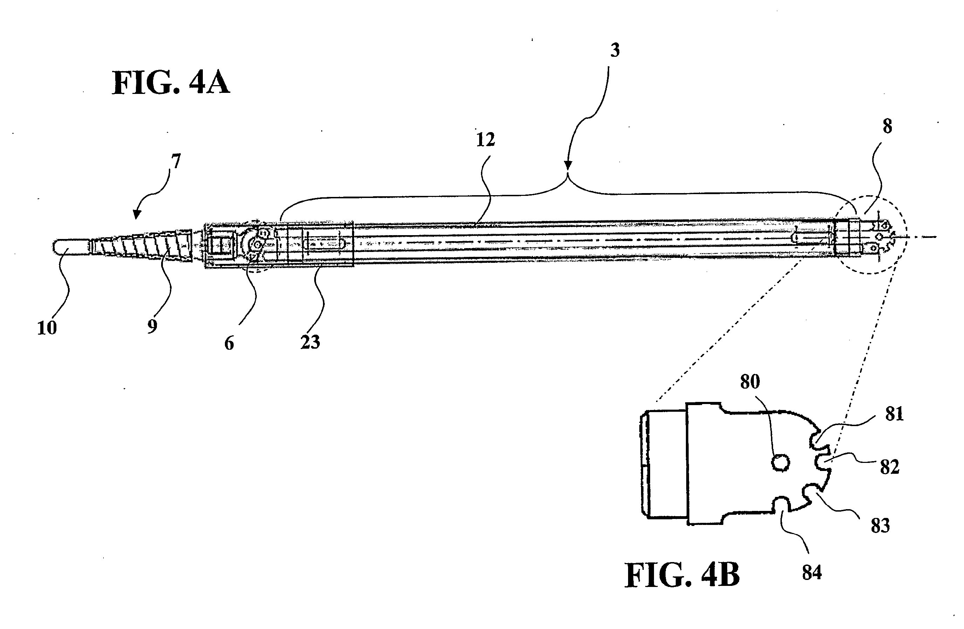

This joint is traversed overall by three first aligned pins 80, 90, 91; the two outermost first pins 90, 91, which can be seen in Figures 4 and 5, allow locking with the eyelets 181, 221 of the rods 18, 22, while the first central pin 80 allows attachment to the central tubular body 3, as shown also in Figure 1B where a fully mounted manipulator, ready for use, is shown.

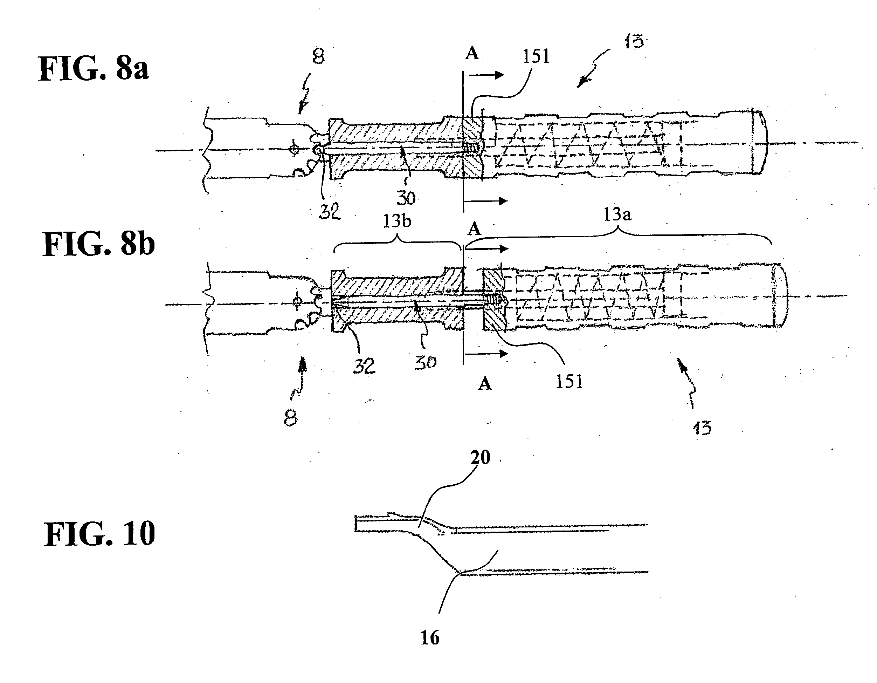

The first control grip 13 is formed as a hollow sleeve and which contains a system (shown in Figures 8a, 8b and 9) for fixing the operative position of the screw 7 in relation to the position of the first control grip 13.

A second central pin 800 allows the connection between the central tubular body 3, the joint 6 and the end portion for front connection to the screw 7, while second external pins 900, 910 allow attachment of the joint 6 to eyelets 181, 221 at the end of the rods 18, 22.

The system, operating via a spring 15, is described herein below with partial reference to Figures 8a, 8b, 9 and 3.

The first control grip 13 houses inside slidingly a locking pin 30 made up of a pin body 31, a tip 32 and a base 33.

A spring 15, also present in the first control grip 13, exerts a force on a block 151, integral with the base 33 and also exerts, therefore, pressure on the pin 30, preventing its longitudinal displacement. The tip 32, in the locked pin condition, is housed in one of the cavities 81, 82, 83 or 84 of the second joint 8 in order to keep the first control grip 13 and the screw 7 in relative fixed positions.

The spring locking system allows the release of the locking pin 30 whenever an operator exerts a pulling force to move a mobile end portion 13A away from an initial fixed portion 13B of the first control grip 13, said movement being exerted in the direction of the reference line A-A.

In this way the locking pin 30 translates longitudinally and integrally with the block 151, causing a displacement of the tip 32 towards the first control grip 13 and the corresponding release of the tip 32 from one of the cavities 81, 82, 83 or 84. In this way the reciprocal rotation of the first control grip 13 is consequently permitted in relation to the central tubular body 3 of the manipulator about the pin 80.

There are a fixed number of positions for locking the pin 30 in the joint 8, which depend on the number of notches in the same joint. Naturally the higher the number of notches in the joint 8, the higher the number of relative positions that can be assumed by the first control grip 13 in relation to the screw 7.

According to the angle of inclination required by the screw 7 in order to perform specific operations of mobilisation of the uterus (such as, for example, the aforementioned anteversion, retroversion, lateral movement and elevation of the uterus, etc.), the operator decides on the corresponding angle of inclination of the first control grip 13, said angle varying according to the notch occupied by the tip 32 of the locking pin 30.

While the screw 7 is positioned in the uterus with its threaded body 9, the first control grip 13 which is in the hands of the operator allows control of the movement of the mobilisation rod 1 and, consequently, of the same screw 7.

The angle between the first control grip 13 and the rod 1 is at maximum amplitude equal to 90° upwards or downwards during rotation about the first pin 80. The screw 7 is rotatable in turn about the second pin 800.

According to the cavity 81, 82, 83 or 84 which the tip 32 of the locking pin 30 is going to occupy, a different locking position of the first control grip 13 will be defined in relation to the screw 7.

Thanks to the articulated quadrilateral 14, the control grip 13 and the screw 7 are coplanar.

The mobilisation rod 1, according to the invention, is used individually to mobilise internal parts of a patient's body, more particularly for the mobilisation of the uterus during operations involving examination of the pelvic viscera.

In a laparoscopic hysterectomy operation, the mobilisation rod 1, via the joint 6, the central body 3 and the joint 8, transmits a movement from the first control grip 13 to the screw 7 and serves to block the uterus to allow execution of the various stages of the operation.

The rod has the advantageous feature of being made as a single part; the only interchangeable part, according to the hysterometry, is the screw 7.

In a laparoscopic hysterectomy operation, the aforementioned complementary unit 2 has to be fitted onto the mobilisation rod 1 but only at the time when the actual colpectomy operation starts. During this stage the use of the two instruments is necessarily combined.

The complementary unit 2 is shown, broken down into its component parts, in Figure 1C and, together with the rod 1, in Figures 1A and 1B.

It comprises a central sleeve 16, with a removable sealing cup 19, which has at a first end an appendage 20 with an isolating portion 21, and at a second end a grip 17.

The sleeve 16 is preferably made of a plastic material or the like, for example of the type known commercially by the name UDEL 1.700 - 7407, and has a hollow cylindrical shape. The shape of the isolating portion 21 of the appendage 20 is anatomically designed to allow easier rotation on the neck of the uterus and the visualisation of the vaginal fornices and is preferably made of ceramic material (of the type known commercially by the name BX-9 Al₂O₃ 99.7%) or a hard material, non-conductive and resistant to high temperatures. It is attached to the appendage 20, for example by means of force fit or other fixing systems.

The removable sealing cup 19 preferably has an ogival shape, is preferably made of silicone and serves to ensure that the pneumoperitoneum is maintained. At the opposite end of the sleeve 16, in relation to the appendage 20, is the second grip 17 suitable for being attached to the sleeve 16 for guiding the movements thereof.

The second grip 17 is preferably made of nylon with inserts of threaded stainless steel, suitable for restraining a screw for attaching the grip on the central sleeve 16.

The complementary unit 2 can be dismantled into its component parts described, allowing a convenient, simple and fast cleaning thereof; as a result the replacement of damaged or worn parts is also easy to carry out.

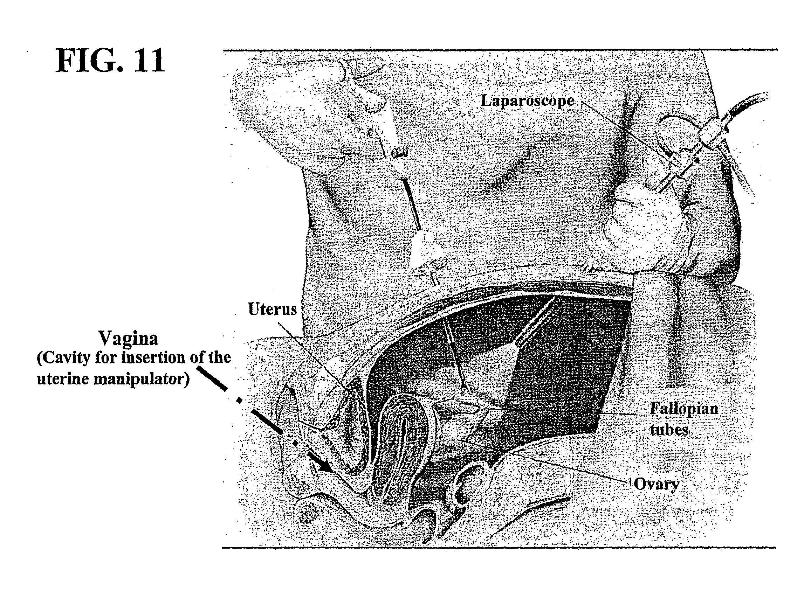

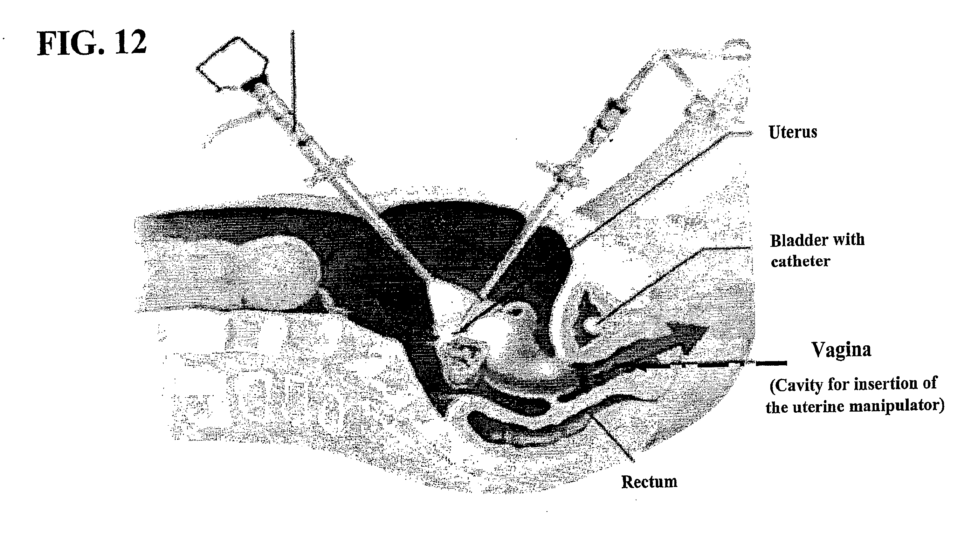

From that which has been described hitherto it is possible to understand the working mode of the uterine manipulator, considered thus far, which will be described by referring generally to Figures 11 and 12 with special reference to a laparoscopic hysterectomy operation, without thereby limiting the sphere of use of the devices described which can be applied to other types of surgical methods; the mobilisation rod 1 can in fact be used for various types of internal examinations, in particular of the pelvic viscera, not jointly with the complementary unit 2.

The laparoscopic hysterectomy operation, with the aid of the uterine manipulator 100, takes place in two different stages:

### - First stage: isolation of the uterus

In this stage the uterus is isolated from the ligaments which connect it to bordering tissues; in particular the round ligaments, broad ligaments and uterosacral ligaments are cut.

For this purpose only the mobilisation rod 1 of the manipulator is used, in accordance with the present invention; as a result the central sleeve 16, fitted with the removable sealing cup 19, is not used in this stage of the operation.

The rod 1, after being fitted with the cap 23 at the joint 6 to avoid the passage of air in the slits used for washing, is inserted, without the complementary unit 2 and with its tip 10, in the cervical channel and in the uterine cavity via the vaginal route (denoted by the arrows in Figures 11 and 12) .

The threaded body 9 of the screw 7 is rotated inside the uterus; this rotation is caused by a corresponding rotation of the mobilisation rod 1, transmitted by an operator, via the first grip 13. In this way the screw 7 is anchored to the neck of the uterus. In this operation, screw 7 and control grip 13 are aligned.

Once the angle of inclination required by the screw 7 in order to perform specific operations for mobilising the uterus has been decided, the operator releases the locking pin 30 by means of the system with spring 15, exerting a pulling force to move the mobile end portion 13A away from the fixed first portion 13B, said movement being performed in the direction of the reference line A-A.

Once the inclination of the first grip 13 in relation to the manipulator axis has been established, that is to say once it has been established which of cavities 81, 82, 83 or 84 will receive the tip 32 of the locking pin 30, the spring 15 of the grip is released, allowing the locking pin 30 to occupy the prechosen cavity, constraining the rotational movement of the first grip 13 with the rotational movement of the screw 7.

The first control grip 13 is moved to displace the uterus and aid the operator in performing the various associated stages of the total hysterectomy by laparoscopy, such as cutting of the uterus connection ligaments from the surrounding tissues mentioned above, a cut which is made by means of an appropriate scalpel, usually an electric scalpel, inserted in the patient's abdomen through an opening formed in an appropriate location.

The rod is moved to allow the manoeuvres of:
- Anteversion and retroversion of the uterus that allow respectively good exposure of the rear wall of the uterus and the uterosacral ligaments and the rear wall of the uterus and the uterovescical fold, making cutting of the broad ligaments simpler and safer.
- Lateral movement of the uterus that allows more convenient cutting of the round ligaments.
- Elevation of the uterus that allows more convenient cutting of the uterosacral ligaments.

### - Second stage: colpectomy

In the second stage denoted by the term "colpectomy", that is to say effective removal of the uterus, the complementary unit 2 is inserted on the rod 1 on the side of the first control grip 13, therefore without removing the rod 1 after its prior positioning at the neck of the uterus. This complementary unit 2 allows, during the final stages of the operation, visualisation of the vaginal fornices.

The convenience of inserting the complementary unit 2 only when needed allows easier mobilisation of the uterus, during the stages prior to the operation.

The uterine manipulator 100 assumes at this time a configuration that is conceptually similar to that of the Clermont-Ferrand manipulator.

The colpectomy is preferably carried out in a circular manner via monopolar cutting currents emitted by an electric scalpel and is guided by the isolating portion 21 of the appendage 20 which avoids dispersion of the cutting current used. The removable sealing cup 19, positioned at the entrance of the vagina, prevents the release of CO2 (used for creating the pneumoperitoneum necessary for the operation).

The uterus, once detached completely from the surrounding tissues, is extracted through the vaginal cavity.

The manipulator according to the invention has a series of undoubted advantages:
- it has a mobilisation rod formed as a single part and easy to assemble, only requiring the choice of the tip depending on the hysterometry;
- it has a low weight (approximately 400g), which represents considerable progress in relation to the more recent background art;
- it is ergonomic, comparable to the natural extension of the arm of the operator who responds promptly to the needs of mobilisation of the uterus;
- it is very easy to treat both for cleaning and washing and sterilisation, given the easy assembly thereof;
- and it is easy to position and use and fast to assemble, also when the mobilisation rod is fitted with the complementary unit;
- it allows smaller dilation of the vagina for insertion, compared to the prior art.

## Claims

1. Uterine manipulator (100) comprising:
- a mobilisation rod (1) comprising a central tubular body (3) connected in an articulated manner at one end to a first control grip (13) and at the other end to a screw (7) and means for transmitting commands from said first control grip (13) to said screw (7);
- a complementary unit (2) comprising a sleeve (16) suitable for being associated coaxially and slidingly with said central tubular body (3) of said mobilisation rod (1) for guiding its movement, an extractable sealing cup (19) which is fittable coaxially on said sleeve (16) and an appendage (20) integral with the end of said sleeve (16) adjacent to said screw (7);
wherein said command transmission means comprise:
- a first joint (6) for the connection of said central tubular body (3) with said screw (7);
- a second joint (8) connecting said central tubular body (3) to said first control grip (13);
- an articulated connection between said first joint (6) and second joint (8) so that each movement of inclination of said first control grip around said second joint (8) corresponds to a movement of inclination of said screw (7) around said first joint (6);
- a locking system for fixing the operative position of said screw (7) arranged inside said first control grip (13) and comprising a locking pin (30) housed slidingly inside said first control grip (13) and pushed by a spring (15) for engaging selectively in one of a plurality of notches (81, 82, 83, 84) in order to keep the control grip and the screw in a selected angular position.

2. Manipulator according to Claim 1 wherein the articulated connection comprises an articulated quadrilateral (14).

3. Manipulator according to Claim 2 wherein said articulated quadrilateral (14) comprises parallel rods (18, 22) of equal length, terminating at the ends with respective first eyelets (181, 221) and second eyelets (182, 222).

4. Manipulator according to one of the previous claims wherein said joints (6, 8) comprise respectively a first central pin (800) and a second central pin (80) and first external pin (900, 910) and second external pin (90, 91), said first pins (80, 90, 91) being aligned with each other on said joint (8) and said second pins (800, 900, 910) being aligned with each other on said joint (6).

5. Manipulator according to Claims 3 and 4 wherein said first eyelets (181, 221) are engaged by said first external pins (900, 910) and said second eyelets (182, 222) are engaged by said second external pins (90, 91), the coupling between eyelets and pins allowing the movement of said rods (18, 22) as a result of the rotation of one of said joints (6, 8).

6. Manipulator according to Claim 1 wherein said screw (7) is made up of a threaded body (9) with a rounded tip (10).

7. Manipulator according to Claim 1 wherein the central tubular body (3) comprises a fork-shaped structure (40) supporting said second joint (8), said fork-shaped structure (40) comprising a first branch (41) and a second branch (42), the second branch (42) consisting of a half disk with said notches (81, 82, 83, 84).

8. Manipulator according to Claim 1 wherein the first control grip (13) comprises an initial fixed portion (13B) and a mobile end portion (13A) connected to the locking pin (30), the movement of the mobile end portion away from the initial fixed portion (13B) disengaging the locking system.

9. Manipulator according to Claim 1 wherein the locking pin (30) is formed by a pin body (31), a tip (32) and a base (33), said base (33) being fixed in a block (151) on which the spring (15) exerts a force for engaging the tip (32) in one of the notches.

10. Manipulator according to one of the previous claims wherein said complementary unit (2) comprises a second grip (17) suitable for controlling the movement of said uterine manipulator.

11. Manipulator according to one of the previous claims wherein said appendage (20) has an isolating portion (21).

12. Mobilisation rod (1) comprising a central tubular body (3) connected in an articulated manner at one end to a first control grip (13) and at the other end to a screw (7) and means for transmitting commands from said first control grip (13) to said screw (7), wherein said command transmission means comprise:
- a first joint (6) for the connection of said central tubular body (3) with said screw (7);
- a second joint (8) for connecting said central tubular body (3) to said first control grip (13);
- an articulated connection between said first joint (6) and second joint (8) so that each movement of inclination of said first control grip around said second joint (8) corresponds to a movement of inclination of said screw (7) around said first joint (6); **characterised by**
- a locking system for fixing the operative position of said screw (7) arranged inside said first control grip (13) and comprising a locking pin (30) housed slidingly inside said first control grip (13) and pushed by a spring (15) for engaging selectively in one of a plurality of notches (81, 82, 83, 84) in order to keep the control grip and the screw in a selected angular position.

13. Mobilisation rod according to Claim 12 wherein the articulated connection comprises an articulated quadrilateral (14).

14. Mobilisation rod according to Claim 13 wherein said articulated quadrilateral (14) comprises parallel rods (18, 22) of equal length, terminating at the ends with respective first eyelets (181, 221) and second eyelets (182, 222).

15. Mobilisation rod according to Claim 12 wherein said joints (6, 8) comprise respectively a first central pin (800) and a second central pin (80) and first external pin (900, 910) and second external pin (90, 91), said first pins (80, 90, 91) being aligned with each other on said joint (8) and said second pins (800, 900, 910) being aligned with each other on said joint (6).

16. Mobilisation rod according to Claims 14 and 15 wherein said first eyelets (181, 221) are engaged by said first external pins (900, 910) and said second eyelets (182, 222) are engaged by said second external pins (90, 91), the coupling between eyelets and pins allowing the movement of said rods (18, 22) as a result of the rotation of one of said joints (6, 8).

17. Mobilisation rod according to Claim 12 wherein said screw (7) is made up of a threaded body (9) with a rounded tip (10).

18. Mobilisation rod according to Claim 12 wherein the central tubular body (3) comprises a fork-shaped structure (40) supporting said second joint (8), said fork-shaped structure (40) comprising a first branch (41) and a second branch (42), the second branch (42) consisting of a half disk with said notches (81, 82, 83, 84).

19. Mobilisation rod according to Claim 12 wherein the first control grip (13) comprises an initial fixed portion (13B) and a mobile end portion (13A) connected to the locking pin (30), the movement of the mobile end portion away from the initial fixed portion (13B) disengaging the locking system.

20. Mobilisation rod according to Claim 12 wherein the locking pin (30) is formed by a pin body (31), a tip (32) and a base (33), said base (33) being fixed in a block (151) on which the spring (15) exerts a force for engaging the tip (32) in one of the notches.

## Patentansprüche

1. Uterusmanipulator (100) umfassend:
Einen Mobilisationsstab (1) umfassend einen mittigen Röhrenkörper (3), der an einem Ende gelenkig mit einem ersten Steuergriff (13) und an dem anderen Ende mit einer Schraube (7) verbunden ist, sowie Mittel zum Übertragen von Befehlen von dem ersten Steuergriff (13) an die Schraube (7),
eine Komplementäreinheit (2) umfassend eine Muffe (16), die geeignet ist, mittig und gleitend mit dem mittigen Röhrenkörper (3) des Mobilisationsstabes (1) verbunden zu werden, um seine Bewegung zu führen, sowie eine extrahierbare Abdichtung (19), die mittig auf der Muffe (16) angeordnet werden kann, und einen Fortsatz (20), der mit dem Ende der Muffe (16) einstückig verbunden ist und an die Schraube (7) angrenzt,
worin das Mittel zum Übertragen von Befehlen umfasst:
Ein erstes Gelenk (6) für die Verbindung des mittigen Röhrenkörpers (3) mit der Schraube (7),
ein zweites Gelenk (8), das den mittigen Röhrenkörper (3) mit dem ersten Steuergriff (13) verbindet,
eine Gelenkverbindung zwischen dem ersten Gelenk (6) und dem zweiten Gelenk (8), so dass jede Neigungsbewegung des ersten Steuergriffes um das zweite Gelenk (8) einer Neigungsbewegung der Schraube (7) um das erste Gelenk (6) entspricht,
ein in dem ersten Steuergriff (13) angeordnetes Arretierungssystem zum Arretieren der Betriebsposition der Schraube (7), umfassend einen Arretierbolzen (30), der gleitbar in dem ersten Steuergriff (13) untergebracht ist und durch eine Feder (15) zum wahlweisen Eingreifen in eine von mehreren Rasten (81, 82, 83, 84) gedrückt wird, um den Steuergriff und die Schraube in einer bestimmten Winkelposition zu halten.

2. Manipulator nach Anspruch 1, worin die Gelenkverbindung ein Gelenkviereck (14) umfasst.

3. Manipulator nach Anspruch 2, worin das Gelenkviereck (14) parallele Stäbe (18, 22) gleicher Länge umfasst, die an ihren Enden mit entsprechenden ersten Ösen (181, 221) und zweiten Ösen (182, 222) enden.

4. Manipulator nach einem der vorstehenden Ansprüche, worin die Gelenke (6, 8) jeweils einen ersten mittigen Stift (800) und einen zweiten mittigen Stift (80) und einen ersten äußeren Stift (900, 910) und einen zweiten äußeren Stift (90, 91) umfassen, worin die ersten Stifte (80, 90, 91) miteinander an dem Gelenk (8) ausgerichtet sind und die zweiten Stifte (800, 900, 910) miteinander an dem Gelenk (6) ausgerichtet sind.

5. Manipulator nach einem der Ansprüche 3 und 4, worin die ersten Ösen (181, 221) durch die ersten äußeren Stifte (900, 910) belegt sind, und die zweiten Ösen (182, 222) durch die zweiten äußeren Stifte (90, 91) belegt sind, worin die Kopplung zwischen Ösen und Stiften eine Bewegung der Stäbe (18, 22) als Folge der Drehung eines der Gelenke (6, 8) ermöglicht.

6. Manipulator nach Anspruch 1, worin die Schraube (7) aus einem gewindeten Körper (9) mit abgerundeter Spitze (10) besteht.

7. Manipulator nach Anspruch 1, worin der mittige Röhrenkörper (3) eine gabelförmige Struktur (40) umfasst, die das zweite Gelenk (8) stützt, worin die gabelförmige Struktur (40) einen ersten Ableger (41) und einen zweiten Ableger (42) umfasst, worin der zweite Ableger (42) aus einer Halbscheibe mit den Rasten (81, 82, 83, 84) besteht.

8. Manipulator nach Anspruch 1, worin der erste Steuergriff (13) einen anfänglich arretierten Bereich (13B) und einen beweglichen Endbereich (13A) umfasst, der mit dem Arretierbolzen (30) verbunden ist, worin eine Bewegung des beweglichen Endbereichs (13A) weg von dem anfänglich arretierten Bereich (13B) das Arretierungssystem löst.

9. Manipulator nach Anspruch 1, worin der Arretierbolzen (30) durch einen Bolzenkörper (31), eine Spitze (32) und eine Basis (33) gebildet wird, worin die Basis (33) in einem Block (151) fixiert vorliegt, auf den die Feder (15) eine Kraft zum Eingreifen der Spitze (32) in eine von den Rasten ausübt.

10. Manipulator nach einem der vorstehenden Ansprüche, worin die Komplementäreinheit (2) einen zweiten Griff (17) umfasst, der geeignet ist, die Bewegung des Uterusmanipulators zu steuern.

11. Manipulator nach einem der vorstehenden Ansprüche, worin der Fortsatz (20) einen Isolierungsbereich (21) aufweist.

12. Mobilisationsstab (1) umfassend einen mittigen Röhrenkörper (3), der an einem Ende gelenkig mit einem ersten Steuergriff (13) und an dem anderen Ende mit einer Schraube (7) verbunden ist, sowie Mittel zum Übertragen von Befehlen von dem ersten Steuergriff (13) an die Schraube (7), worin das Mittel zum Übertragen von Befehlen umfasst:
Ein erstes Gelenk (6) für die Verbindung des mittigen Röhrenkörpers (3) mit der Schraube (7),
ein zweites Gelenk (8), das den mittigen Röhrenkörper (3) mit dem ersten Steuergriff (13) verbindet,
eine Gelenkverbindung zwischen dem ersten Gelenk (6) und dem zweiten Gelenk (8), so dass jede Neigungsbewegung des ersten Steuergriffes um das zweite Gelenk (8) einer Neigungsbewegung der Schraube (7) um das erste Gelenk (6) entspricht, **gekennzeichnet durch**
ein in dem ersten Steuergriff (13) angeordnetes Arretierungssystem zum Arretieren der Betriebsposition der Schraube (7), umfassend einen Arretierbolzen (30), der gleitbar in dem ersten Steuergriff (13) untergebracht ist und **durch** eine Feder (15) zum wahlweisen Eingreifen in eine von mehreren Rasten (81, 82, 83, 84) gedrückt wird, um den Steuergriff und die Schraube in einer bestimmten Winkelposition zu halten.

13. Mobilisationsstab nach Anspruch 12, worin die Gelenkverbindung ein Gelenkviereck (14) umfasst.

14. Mobilisationsstab nach Anspruch 13, worin das Gelenkviereck (14) parallele Stäbe (18, 22) gleicher Länge umfasst, die an ihren Enden mit entsprechenden ersten Ösen (181, 221) und zweiten Ösen (182, 222) enden.

15. Mobilisationsstab nach Anspruch 12, worin die Gelenke (6, 8) jeweils einen ersten mittigen Stift (800) und einen zweiten mittigen Stift (80) und einen ersten äußeren Stift (900, 910) und einen zweiten äußeren Stift (90, 91) umfassen, worin die ersten Stifte (80, 90, 91) miteinander an dem Gelenk (8) ausgerichtet sind und die zweiten Stifte (800, 900, 910) miteinander an dem Gelenk (6) ausgerichtet sind.

16. Mobilisationsstab nach einem der Ansprüche 14 und 15, worin die ersten Ösen (181, 221) durch die ersten äußeren Stifte (900, 910) belegt sind und die zweiten Ösen (182, 222) durch die zweiten äußeren Stifte (90, 91) belegt sind, worin die Kopplung zwischen Ösen und Stiften eine Bewegung der Stäbe (18, 22) als Folge der Drehung eines der Gelenke (6, 8) ermöglicht.

17. Mobilisationsstab nach Anspruch 12, worin die Schraube (7) aus einem gewindeten Körper (9) mit abgerundeter Spitze (10) besteht.

18. Mobilisationsstab nach Anspruch 12, worin der mittige Röhrenkörper (3) eine gabelförmige Struktur (40) umfasst, die das zweite Gelenk (8) stützt, worin die gabelförmige Struktur (40) einen ersten Ableger (41) und einen zweiten Ableger (42) umfasst, worin der zweite Ableger (42) aus einer Halbscheibe mit den Rasten (81, 82, 83, 84) besteht.

19. Mobilisationsstab nach Anspruch 12, worin der erste Steuergriff (13) einen anfänglich arretierten Bereich (13B) und einen beweglichen Endbereich (13A) umfasst, der mit dem Arretierbolzen (30) verbunden ist, worin eine Bewegung des beweglichen Endbereichs (13A) weg von dem anfänglich arretierten Bereich (13B) das Arretierungssystem löst.

20. Mobilisationsstab nach Anspruch 12, worin der Arretierbolzen (30) durch einen Bolzenkörper (31), eine Spitze (32) und eine Basis (33) gebildet wird, worin die Basis (33) in einem Block (151) fixiert vorliegt, auf den die Feder (15) eine Kraft zum Eingreifen der Spitze (32) in eine von den Rasten ausübt.

## Revendications

1. Manipulateur utérin (100) comprenant :
une tige de mobilisation (1) comprenant un corps tubulaire central (3) raccordé, d'une manière articulée, au niveau d'une extrémité, à une première poignée de commande (13) et au niveau de l'autre extrémité, à une vis (7) et des moyens pour transmettre des commandes de ladite première poignée de commande (13) à ladite vis (7) ;
une unité complémentaire (2) comprenant un manchon (16) approprié pour être associé de manière coaxiale et coulissante avec ledit corps tubulaire central (3) de ladite tige de mobilisation (1) pour guider son mouvement, un cupule d'étanchéité extractible (19) qui peut être montée de manière coaxiale sur ledit manchon (16) et un appendice (20) solidaire de l'extrémité dudit manchon (16) adjacent à ladite vis (7) ;
dans lequel lesdits moyens de transmission de commande comprennent :
un premier joint (6) pour le raccordement dudit corps tubulaire central (3) avec ladite vis (7) ;
un second joint (8) raccordant ledit corps tubulaire central (3) à ladite première poignée de commande (13) ;
un raccordement articulé entre ledit premier joint (6) et ledit second joint (8) de sorte que chaque mouvement d'inclinaison de ladite première poignée de commande autour dudit second joint (8) correspond à un mouvement d'inclinaison de ladite vis (7) autour dudit premier joint (6) ;
un système de verrouillage pour fixer la position opérationnelle de ladite vis (7) agencée à l'intérieur de ladite première poignée de commande (13) et comprenant une broche de verrouillage (30) logée de manière coulissante à l'intérieur de ladite première poignée de commande (13) et poussée par un ressort (15) pour se mettre sélectivement en prise dans l'une d'une pluralité d'encoches (81, 82, 83, 84) afin de garder la poignée de commande et la vis dans une position angulaire sélectionnée.

2. Manipulateur selon la revendication 1, dans lequel le raccordement articulé comprend un quadrilatère articulé (14).

3. Manipulateur selon la revendication 2, dans lequel ledit quadrilatère articulé (14) comprend des tiges parallèles (18, 22) de longueur identique, se terminant au niveau des extrémités avec des premiers oeillets (181, 221) et seconds oeillets (182, 222) respectifs.

4. Manipulateur selon l'une des revendications précédentes, dans lequel lesdits joints (6, 8) comprennent respectivement une première broche centrale (800) et une seconde broche centrale (80) et la première broche externe (900, 910) et la seconde broche externe (90, 91), lesdites premières broches (80, 90, 91) étant alignées les unes par rapport aux autres sur ledit joint (8) et lesdites secondes broches (800, 900, 910) étant alignées les unes par rapport aux autres sur ledit joint (6).

5. Manipulateur selon les revendications 3 et 4, dans lequel lesdits premiers oeillets (181, 221) sont mis en prise par lesdites premières broches externes (900, 910) et lesdits seconds oeillets (182, 222) sont mis en prise par lesdites secondes broches externes (90, 91), le couplage entre les oeillets et les broches permettant le mouvement desdites tiges (18, 22) suite à la rotation de l'un desdits joints (6, 8).

6. Manipulateur selon la revendication 1, dans lequel ladite vis (7) est composée d'un corps fileté (9) avec une pointe arrondie (10).

7. Manipulateur selon la revendication 1, dans lequel le corps tubulaire central (3) comprend une structure en forme de fourche (40) supportant ledit second joint (8), ladite structure en forme de fourche (40) comprend une première branche (41) et une seconde branche (42), ladite seconde branche (42) se composant d'un demi disque avec lesdites encoches (81, 82, 83, 84).

8. Manipulateur selon la revendication 1, dans lequel la première poignée de commande (13) comprend une partie fixe initiale (13B) et une partie d'extrémité mobile (13A) raccordée à la broche de verrouillage (30), le mouvement de la partie d'extrémité mobile à distance de la partie fixe initiale (13B) dégageant le système de verrouillage.

9. Manipulateur selon la revendication 1, dans lequel la broche de verrouillage (30) est formée par un corps de broche (31), une pointe (32) et une base (33), ladite base (33) étant fixée dans un bloc (151) sur lequel le ressort (15) exerce une force pour mettre en prise la pointe (32) dans l'une des encoches.

10. Manipulateur selon l'une des revendications précédentes, dans lequel ladite unité complémentaire (2) comprend une seconde poignée (17) appropriée pour commander le mouvement dudit manipulateur utérin.

11. Manipulateur selon l'une des revendications précédentes, dans lequel ledit appendice (20) a une partie isolante (21).

12. Tige de mobilisation (1) comprenant un corps tubulaire central (3) raccordé, d'une manière articulée, au niveau d'une extrémité, à une première poignée de commande (13) et au niveau de l'autre extrémité, à une vis (7) et des moyens pour transmettre des commandes de ladite première poignée de commande (13) à ladite vis (7), dans laquelle lesdits moyens de transmission de commande comprennent :
un premier joint (6) pour le raccordement dudit corps tubulaire central (3) avec ladite vis (7) ;
un second joint (8) pour le raccordement dudit corps tubulaire central (3) à ladite première poignée de commande (13) ;
un raccordement articulé entre ledit premier joint (6) et le second joint (8) de sorte que chaque mouvement d'inclinaison de ladite première poignée de commande autour dudit second joint (8) correspond à un mouvement d'inclinaison de ladite vis (7) autour dudit premier joint (6) ; **caractérisée par** :
un système de verrouillage pour fixer la position opérationnelle de ladite vis (7) agencée à l'intérieur de ladite première poignée de commande (13) et comprenant une broche de verrouillage (30) logée de manière coulissante à l'intérieur de ladite première poignée de commande (13) et poussée par un ressort (15) pour se mettre sélectivement en prise dans l'une d'une pluralité d'encoches (81, 82, 83, 84) afin de maintenir la poignée de commande et la vis dans une position angulaire sélectionnée.

13. Tige de mobilisation selon la revendication 12, dans laquelle le raccordement articulé comprend un quadrilatère articulé (14).

14. Tige de mobilisation selon la revendication 13, dans laquelle ledit quadrilatère articulé (14) comprend des tiges parallèles (18, 22) de longueur identique, se terminant au niveau des extrémités avec des premiers oeillets (181, 221) et des seconds oeillets (182, 222) respectifs.

15. Tige de mobilisation selon la revendication 12, dans laquelle lesdits joints (6, 8) comprennent respectivement une première broche centrale (800) et une seconde broche centrale (80) et la première broche externe (900, 910) et la seconde broche externe (90, 91), lesdites premières broches (80, 90, 91) étant alignées les unes par rapport aux autres sur ledit joint (8) et lesdites secondes broches (800, 900, 910) étant alignées les unes par rapport aux autres sur ledit joint (6).

16. Tige de mobilisation selon les revendications 14 et 15, dans laquelle lesdits premiers oeillets (181, 221) sont mis en prise par lesdites premières broches externes (900, 910) et lesdits seconds oeillets (182, 222) sont mis en prise par lesdites secondes broches externes (90, 91), le couplage entre les oeillets et les broches permettant le mouvement desdites broches (18, 22) suite à la rotation de l'un desdits joints (6, 8).

17. Tige de mobilisation selon la revendication 12, dans laquelle ladite vis (7) est composée d'un corps fileté (9) avec une pointe arrondie (10).

18. Tige de mobilisation selon la revendication 12, dans laquelle le corps tubulaire central (3) comprend une structure en forme de fourche (40) supportant ledit second joint (8), ladite structure en forme de fourche (40) comprenant une première branche (41) et une seconde branche (42), la seconde branche (42) se composant d'un demi-disque avec lesdites encoches (81, 82, 83, 84).

19. Tige de mobilisation selon la revendication 12, dans laquelle la première poignée de commande (13) comprend une partie fixe initiale (13B) et une partie d'extrémité mobile (13A) raccordée à la broche de verrouillage (30), le mouvement de la partie d'extrémité mobile à distance de la partie fixe initiale (13B) dégageant le système de verrouillage.

20. Tige de mobilisation selon la revendication 12, dans laquelle la broche de verrouillage (30) est formée par un corps de broche (31), une pointe (32) et une base (33), ladite base (33) étant fixée dans un bloc (151) sur lequel le ressort (15) exerce une force pour mettre en prise la pointe (32) dans l'une des encoches.
